# EUROPEAN PATENT APPLICATION

(11) **EP 2 216 639 A1**
(43) Date of publication of application: **11.08.2010**
(21) Application number: 08853229.6
(22) Date of filing: 28.10.2008
(51) Int. Cl.: G01N 11/04, G01N 33/49

(54) **BLOOD FLUIDITY MEASUREMENT APPARATUS AND BLOOD FLUIDITY MEASUREMENT METHOD**

(30) Priority: 28.11.2007 JP 2007306975
(71) Applicant: Konica Minolta Opto, Inc., Hachioji-shi Tokyo 192-8505 (JP)
(72) Inventor: ICHITANI, Shuji, Hino-shi Tokyo 191-8511 (JP); TOYAMA, Osamu, Hino-shi Tokyo 191-8511 (JP)
(74) Representative: Tyson, Robin Edward
(86) International application number: PCT/JP2008/069529
(87) International publication number: WO 2009/069418

(57) **Abstract**

Deformability of blood cells is quantified in a short time. A blood fluidity measurement apparatus is provided with a TV camera (6) which photographs a stream of blood in either two areas of the internal area (A), entrance area (B), and exit area (C) of a gate (30), an image processing part (7) which calculates the velocity of the blood cells contained in the blood from the image taken by the TV camera (6), and a deformability calculation means (81) which calculates the deformability of the blood cells a blood fluidity from the velocity.

## Description

### TECHNICAL FIELD

The present invention relates to a blood fluidity measurement apparatus and a blood fluidity measurement method.

### BACKGROUND TECHNOLOGY

In recent years, along with increasing awareness of health, blood fluidity has been paid attention as a health barometer. The fluidity is also called as the degree of smoothness, and it means that the higher the fluidity or the smoothness is the better in health.

As a method for investigating the above blood fluidity, it has been known that, as described for example in Patent Document 1, blood is allowed to pass through a filter with fine grooves, and the time required for passing through the filter is measured. Further, it is also possible for the method of Patent Document 1 to grasp visually the blood fluidity by observing with a camera blood cells passing through the filter using a filter substrate made of a transparent glass. Further, as described in Non Patent Document 1, a method for evaluating a deformability (ease of deformation) of blood cells which is a representative parameter ofblood fluidity, by an image capturing deforming blood in a filter has been proposed.

These methods are methods for investigating mainly fluidity of blood cells. For example, in Patent Document 2, a method for capturing movements of white blood cells in an adherence phenomenon in which white blood cells adhere to blood veins which is particular to white blood cells. In this method, a moving velocity and so on ofblood cells in a process to adhere to blood veins can be calculated by digital processing the image capturing the movement of the white blood cell.
Patent Document 1: Japanese Patent No. 2685544
Patent Document 2: Japanese Patent Application Publication No. 2001-264318
Non Patent Document 1: Tsutomu Tajikawa et al. "Visualization and Observation of Deformation of Human Red Blood Cell Passing Through a Micro-channel Array as a Model of Human blood Capillary", pages 84-91, December 2005, Vol.25 No.12 Transaction of the Visualization Society of Japan.

### DISCLOSURE OF INVENTION

### PROBLEMS TO BE SOLVED BY THE INVENTION

However, with the method described in Patent Document 1, it is not possible to make a quantitative evaluation for the blood fluidity, especially for deformability of blood. With the technology described in Patent Document 2 regarding to the movement of white blood cells, it is not possible to quantify the movement of white blood cells either. Further, with the technology of Non Patent Document 1, the operation of the quantification is time consuming because the operation is performed each by each for each blood cell although the quantification of the deformability is tried.

The present invention has been achieved in consideration of the above problems, and it is an object of the invention to provide a blood fluidity measurement apparatus and a blood fluidity measurement method, which can evaluate the deformability ofblood cells which is a representative parameter of blood fluidity, in a short time.

### MEANS TO SOLVE THE PROBLEMS

To solve the above-described problems, the invention described in Claim 1 is **characterized in that** a blood fluidity measurement apparatus which measures a blood fluidity by flowing blood in a plurality of gates each of which is formed to be narrower than a blood diameter, comprises:
an imaging means which takes an image of blood flow in each of any two areas of an internal area, an inlet area and an outlet area of the gate, or in each internal area of the plurality of the gates;
a velocity calculation means which calculates a velocity of blood cells in the blood by the image taken by the imaging means; and
a deformability calculation means which calculates a deformability ofblood cells as a blood fluidity, by the velocity.

The invention described in Claim 2 is the blood fluidity measurement apparatus described in Claim 1, **characterized in that:**
the imaging means takes an image ofblood flow in any two areas of an internal area, an inlet area and an outlet area of the gate,
the velocity calculation means calculates a velocity of blood cells of each of the two areas at least at one gate; and
the deformability calculation means calculates a difference or a ration of velocities as a deformability of blood cells.

The invention described in Claim 3 is the blood fluidity measurement apparatus described in Claim 1, **characterized in that:**
the imaging means takes an image of blood flow in each internal area of the plurality of the gates;
the velocity calculation means calculates a velocity of blood cells in each area;
the deformability calculation means calculates a number of gates in which the velocity is less than or equal to a predetermined threshold value, as a deformability of blood.

The invention described in Claim 4 is the blood fluidity measurement apparatus described in Claim 1, **characterized in that:**
the imaging means takes an image ofblood flow in each internal area of the plurality of the gates;
the velocity calculation means calculates a velocity of blood cells in each area;
the deformability calculation means calculates a ratio of a number of gates in which the velocity is less than or equal to a predetermined threshold value, to a total number of the gates, as a deformability of blood.

The invention described in Claim 5 is the blood fluidity measurement apparatus described in Claim 3 or Claim 4, **characterized in that:**
the deformability calculation means employs the a half of a velocity ofblood cells in blood when blood having a standard fluidity flows in the internal area, as the predetermined threshold value.

The invention described in Claim 6 is a blood fluidity measurement apparatus described in Claim 3 or Claim 4, **characterized in that**
the deformability calculation means employs 5% of a velocity ofblood cells in blood when blood having a standard fluidity flows in the internal area, as the predetermined threshold value.

The invention described in Claim 7 is a blood fluidity measurement method **characterized in that** the method comprises measuring a blood fluidity using the blood fluidity measurement apparatus described in any one of Claim 1 through 6.

### EFFECTS OF THE INVENTION

According to the invention described in Claim 1, since there are provided an imaging means which takes an image of blood flow in each of any two areas of an internal area, an inlet area and an outlet area of the gate, or in each internal area of the plurality of the gates; a velocity calculation means which calculates a velocity ofblood cells in the blood by the image taken by the imaging means; and a deformability calculation means which calculates a deformability of blood cells as a blood fluidity, by the velocity, therefore, in case of a timing when an image is taken by the imaging means, deformability of all the blood included in the image can be calculated arbitrarily as blood fluidity from the velocity. As a result, compared to conventional cases in which the quantification operation is performed for each blood cell individually, the quantification of deformability ofblood cells can be performed in a shorter time.

### BRIEF DESCRIPTION OF DRAWINGS

Fig.1 is a schematic constitution diagram of a blood fluidity measurement apparatus according to an embodiment of the present invention.
Fig. 2 is a cross section of a filter according to an embodiment of the present invention.
Fig. 3a is a partial top view of a gate according to an embodiment of the present invention, and Fig. 3b is a side view when viewed from a downstream side of the gate.
Fig. 4 is a flow chart of a step in which moving images of blood flow is image processed to investigate fluidity.
Fig. 5 is a figure showing a blood cell flowing in each area of the gate.
Fig. 6 is a figure showing an example of an image of blood flow, in which a gate is clogged.
Fig. 7 is a flow chart of a step in which moving images of blood flow is processed to investigate fluidity.
Fig. 8 is a figure showing a change of a velocity of blood cell in standard blood which flow in an internal area of the gate.

### DESCRIPTION OF ALPHANUMERIC DESIGNATIONS

- 1, 1A, and 1B:: blood fluidity measurement apparatus
- 6, 6A, and 1B:: TV camera (a means for taking an image)
- 7:: Image processing section (a detection means for the state of blood flow)
- 30:: Gate
- A:: Internal area
- B:: Inlet area
- C:: Outlet area
- D:: Speed difference
- L:: Ratio of gate numbers
- N:: Gate number
- R: Velocity ratio
- V_{A}:: Velocity of blood cell at internal area

### PREFERRED EMBODIMENT OF THE PRESENT INVENTION

Embodiments of the present invention will hereinafter be descried with reference to figures.

### [Embodiment of the Present Invention]

First, regarding the blood fluidity measurement apparatus according to the present embodiment, an outline of a constitution thereof will be described

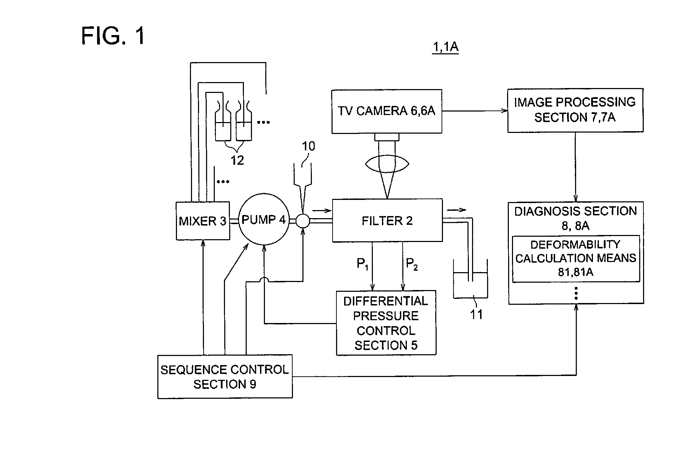

Fig.1 shows a constitution diagram of a blood fluidity measurement apparatus 1 according to an embodiment of the present invention. The blood fluidity measurement apparatus 1 is an apparatus, which introduces blood injected from an inlet 10 into a discharge section 11 through a filter 2, and investigates blood fluidity using information obtained from the above process. The aforesaid apparatus is provided with a TV camera 6 as an imaging means which takes an image of blood flow in the filter 2, an image processing section 7 as a detection means of the state of blood flow to detect the state of blood flow from moving images taken by the above TV camera 6, and a diagnosis section 8 to diagnose the above state of blood flow.

Further, the blood fluidity measurement apparatus 1 not only introduces blood to the filter 2 as it is, but also is provided with a plurality of solution bottles 12 connected to a channel through a mixer 3 for the purpose of introducing blood to the filter 2 after mixing the blood with other solutions such as a physiological salt solution, and a physiological active substance. Blood and so on, which are introduced to the filter 2, are designed so that the desired amount ofblood and others are flowed, by controlling a pump 4 with a differential pressure controlling part 5 to regulate differential pressure in front of and behind the filter 2. The mixer 3, the pump 4, a valve of the inlet 10, and the diagnosis section 8 are integrated and controlled by a sequence control part 9.

Further, the blood fluidity measurement apparatus 1 and a fluidity measurement method which will be described later are not limited specifically, however it is preferable that they are employed in case of calculating a deformability of red blood cells which are hard to clog.

Next, major elements among the constituent elements of the above-described blood fluidity measurement apparatus 1 will be detailed.

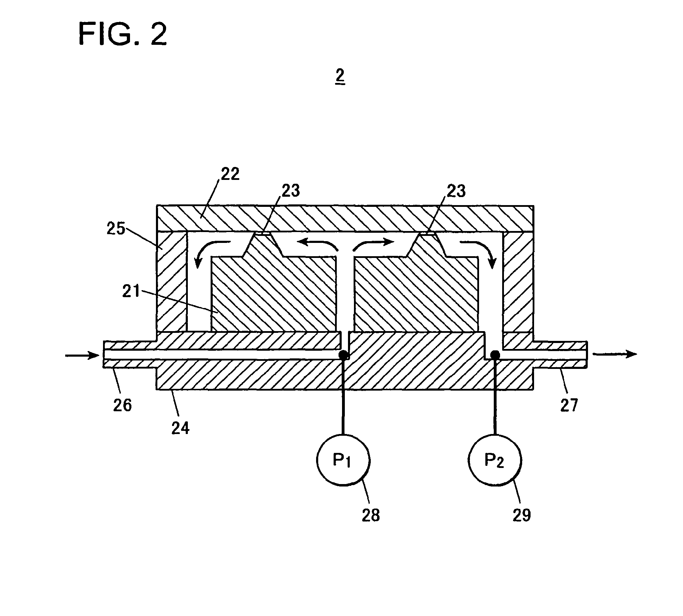

The filter 2 is provided with apertures 23 composed of a group of fine channels between a silicon single crystal substrate 21 and a glass flat board 22, as shown in a cross section of Fig. 2. In order to introduce blood into the above opening 23 and then discharge the blood, a base board 24 and an external cylinder 25 are arranged. The base board 24 has an inlet port 26 and an outlet 27 ofblood, and the blood flows following arrows in the figure. Each pressure in front of and behind the opening 23 is detected by pressure sensors 28 and 29, and pressure signals P1 and P2 caused by the pressures are sent to the differential pressure control part 5 shown in Fig. 1.

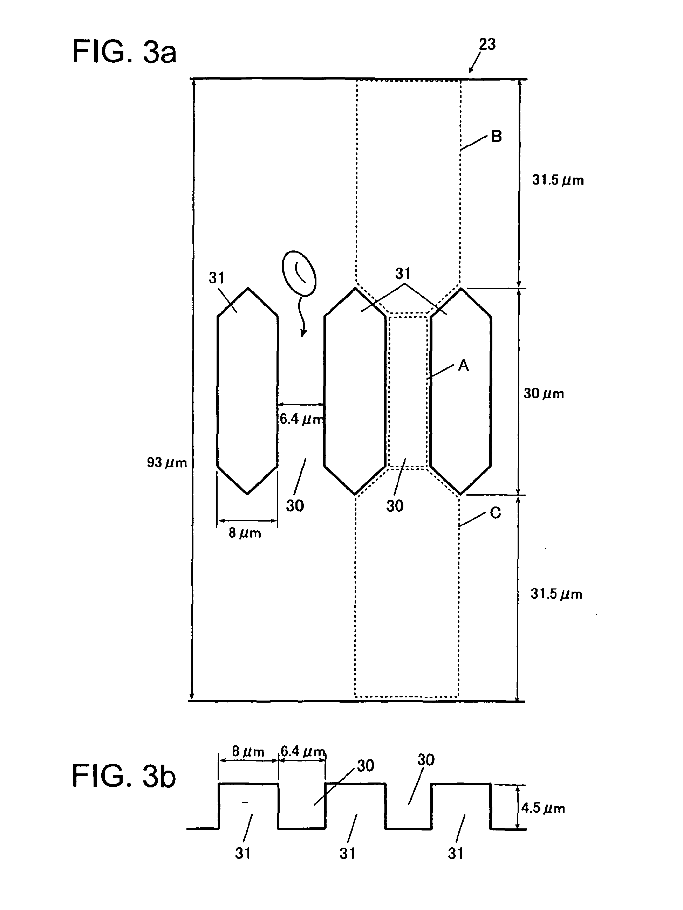

The opening 23 the filter 2 comprises is, as shown in Figs. 3a and 3b, provided with a lot of gates 30 formed as a part sandwiched between two hexagonal banks 31. Fig. 3a is a top view of a part of the opening 23 when viewed from glass flat board 22, and Fig. 3b is a side view of opening 23 when viewed from a downstream side. The upper surface ofbank 31 is made flat, and is joined with the glass flat board 22, not shown in Figs. 3a and 3b. Constituents in blood introduced from the feed port 26, for example blood cells, pass down through the gate 30 of Fig. 3a while being transformed. The above opening 23 is formed in the sizes shown in Figs. 3a and 3b, but is not particularly limited to them. However, the width of the gate 30 (6.4 µ m in Fig. 3a) is required to be smaller than the size of an object, in which transformation is observed, for example a blood cell size of a red blood cell (about 8 µ m).

The TV camera 6 is, for example, a digital CCD camera, and a high-speed camera having enough resolution to take moving images of blood flow. The above TV camera 6 is arranged at the upper part of the filter 2, and takes images, from a side of the glass flat board 22, of blood flow passing through the opening 23. The area of taking an image may, as shown in Fig. 3a, be an area including the internal area A, the inlet area B, and the outlet area C of a plurality of gates 30. However, the area of taking an image may includes any two of areas A through C of at least one gate 30 or may include every internal area A for the plurality of gates 30. It is designed that the moving images ofblood flow obtained by the TV camera 6 can be displayed on a not illustrated display. The above TV camera may be a camera for taking a still image.

The image processing section 7 is provided with an analytical means such as a CPU or a memory means such as a semiconductor memory, and is electrically connected with the TV camera 6. The image processing section 7 processes moving images of blood flow obtained by the TV camera 6, and calculates a velocity of blood cells in blood at each area of areas A through C in the plurality og gates 30. The calculation in this case, in alt least one gate 30, may be performed for any two of areas A through C. The calculated blood velocity can be displayed on a not illustrated display.

A diagnosis section 8 is provided with a deformability calculation means 81, which calculates a deformability ofblood cells by the velocity ofblood cells obtained by the image processing section 7, in addition to an analytical means such as a CPU or a memory means such as a semiconductor memory, and is electrically connected with the image processing section 7. The diagnosis section 8 calculates the deformability ofblood cells such as a ratio of velocities R and velocity difference D which are described later, as a fluidity of blood and diagnoses a degree of health ofblood by the deformability of blood. Further the diagnosis section 8 is provided with data which are necessary for judging the degree of health of blood. The calculated deformability and the diagnosis result can be displayed on a not illustrated display. The diagnosis section 8 may be constituted in an integrated fashion with the image processing section 7 using a PC or other means.

Next, a blood fluidity measurement method via the blood fluidity measurement apparatus 1 according to an embodiment of the present invention will be described.

First, steps until a step of taking an image of blood flow after blood is flowed in the filter 2 will be described.

First, blood for measurement is charged into the inlet 10, and at the same time, a physiological salt solution or others is added to a solution bottle 12, as needed. Then, blood and a physiological salt solution or others (hereinafter, referred to as blood) are flowed to the filter 2 by putting differential pressure on the filter 2, and at the same time, images ofblood flow passing through the gate 30 are taken by the TV camera 6.

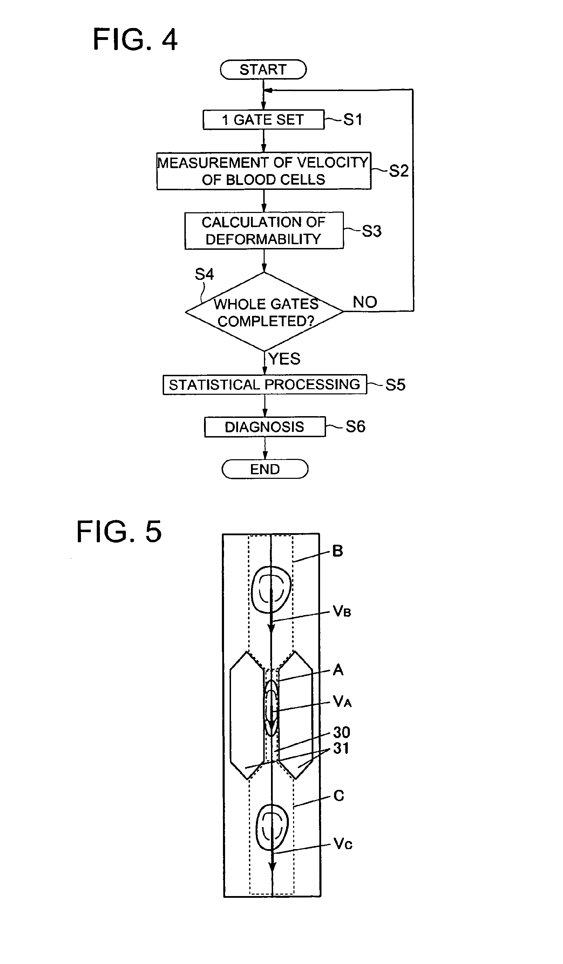

Next, a process, in which images of moving images of blood flow are processed to investigate the fluidity, will be described. This process is executed following steps shown in Fig. 4.

First, one of the gates 30 to be inspected is set (S1). Then the velocity ofblood cells at the set gate 30 is calculated by the image processing section 7(S2).

In calculating the velocity of blood cells, as shown in Fig.5, a velocity V_{A} at internal area A, a velocity V_{B} at internal area B, a velocity V_{C} at internal area C of gate 30 are calculated. At this occasion, it is preferable to calculate the velocity of bloods on the center line of the gate 30. After that, the state of blood flow at the outlet area C of the gate 30 is measured (S3). To calculating the above speeds, methods described in Japanese Patent Application Publications No. H2-257931, H6-18539, 2001-264318 and No. 2006-223761 can be used, for example. The velocity to be calculated can be value represented by one blood cell each for each area or can be a mean value of calculated velocities of a plurality of blood cells. For the calculation of velocity, as described above, the calculation of two velocities among velocities V_{A}, V_{B}, V_{C} suffices. It is preferable to calculate V_{A} and V_{B} because it is difficult for the flow of V_{C} to flow in the direction of the centerline of gate 30.

After the above calculation of blood cells (S2 of Fig. 4), the deformability calculation means 81 calculates a deformability ofblood cells as a fluidity of blood (S3). Specifically, a speed ratio R and a speed difference D are calculated. At least one of R₁ =V_{A}/V_{B}, R₂ =V_{A}V_{C}, R₃ =V_{C}/V_{B} as the speed ratio R, and D₁ =V_{A}-V_{B}, D₂ =V_{A}-V_{C}, D₃ =V_{C}/V_{B} as the speed difference D are each calculated. Only either one of the speed ratio R and the speed ratio D may be calculated.

Further as the deformability of the blood cells, a ratio of the speed ratio R and so on with the blood of a standard degree of health may be calculated. In more details, a velocity V_{S}, a speed ratio R_{S} and a speed difference D₁ of standard blood at the internal area A are stored in advance or kept as data, and each of ratios with the standard blood that is at least one of =V_{A}/V_{S}, R/R_{S}, and D/Ds may be calculated, though it is necessary that the speed ratio or the speed difference are calculated for the velocities of the standard blood and the object blood at the same area, when the speed ratio or the speed difference are calculated.

Further as the deformability of the blood cells, a ratio of the speed ratio R and so on with normal saline of which speeds at each of areas A through C of gate 30 should not change may be calculated. In more details, a velocity Vo, a speed ratio R_{O} and a speed difference D_{O} of normal saline at the internal area A are stored in advance or kept as data, and each of ratios with the normal saline that is at least one of =V_{O}/V_{A} and R_{O}/R may be calculated, though it is necessary that the speed ratio R and R_{O} are calculated for the velocities of the normal saline and the object blood at the same area, when the ratio of the speed ratio is calculated.

Further a conversion table of the deformability and speed ratio R obtained by a filtration method is kept as data, and deformability of the object blood may be calculated from the speed ration by using the table. Here, the filtration is a known method which obtains the deformability from changes of pressure.

At a timing when an image is taken by a TV camera 6, the above described deformability can be arbitrarily calculated for the blood cells included in the image by the step of calculation. Therefore the deformability of bloods can be quantified in a short time, without requiring the operation of the deformability calculation for the individual blood cells as the conventional method.

After calculating the deformability, the steps till now (S1 through S3) are performed for all gates 30 (S4), subsequently, a static calculation processing is performed (S5). The processing is, for example, a processing which obtains a representative value for the deformability by calculating the average value for all gates, and is performed at the diagnosis section 8. In ca se when a plurality of types of deformability are calculated, the processing is performed each by each.

Finally, the diagnosis section 8 diagnoses regarding to the deformability (S6). In the diagnosis, the speed ratio R and the speed difference D is compared with the speed ration R_{S} and the speed difference D_{S} of standard blood. If the speed ratio R is larger than the speed ration R_{S} of the standard blood, the blood is superior in the deformability and it can be said that the blood has a higher degree of health than the standard blood. Further, if the speed difference D is larger than the speed difference D_{S} of the standard blood, similarly, the blood is superior in the deformability and it can be said that the blood has a higher degree of health than the standard blood.

Further, in case when the deformability which has been obtained as a ratio with the standard blood is diagnosed, for example, it can be regarded that it is superior when the speed ratio R/R_{S} is larger than 1, and that it is inferior when the speed ratio R/R_{S} is smaller than 1. Further, in case when the deformability which has been obtained as a ratio with the normal saline is diagnosed, for example, it can be regarded that it is superior when the speed ratio is nearer to 1, and that it is inferior when the speed ratio is nearer to 0.

As described above, according to the blood fluidity measurement apparatus 1 of the present embodiment, the above apparatus is provided with the TV camera 6 which takes an image of a flow of the blood at any two areas among areas A through C, the image processing section 7 which calculates the velocity ofblood cells in the blood by the image taken by the TV camera 6, and the deformability calculation means 81 which calculates the deformability ofblood cells as a fluidity of blood, therefore in case of a timing when an image is taken by the TV camera 6, deformability of all the blood included in the image can be calculated arbitrarily as blood fluidity from the velocity. As a result, the quantification operation is not needed to perform for each blood cell individually as the conventional method, the quantification of deformability ofblood cells can be performed in a shorter time.

### [Modified Example of the Embodiment]

Subsequently, an modified example of the blood fluidity measurement apparatus 1 will be described. Any constituent element similar to the above embodiment will be denoted by the same reference numerals, and their descriptions will be omitted.

The blood fluidity measurement apparatus 1A is, as shown in Fig.1, provided with a TV camera 6A in the above-described embodiment, an image processing section 7A, a diagnosis section 8A, and a modified deformability calculation means 81.

The blood fluidity measurement apparatus 1A in the modified example of the embodiment and a fluidity measuring method using the blood fluidity measurement apparatus which will be described later is not particularly limited. However it is desirable to be used for calculating de deformability of white blood cells which are larger in diameter than red blood cells and is easy to clog at the gate 30.

It is fine for TV 6A, when the area of taking picture of the TV 6A includes at least the internal area A of each of the plurality of gates 30. Other locations than this area of talking picture, too, are configured as same as TV 6 of above described embodiment

The image processing section 7A is provided with an analytical means such as CPU and a memory means such as a semiconductor memory, and is electrically connected with the TV camera 6A. The image processing section 7A processes moving images of blood flow obtained by the TV camera 6A, and calculates a velocity of blood cells in blood at each area of areas A in the plurality of gates 30. The calculated blood velocity can be displayed on a not illustrated display. The image processing section 7A is further provided with data of a velocity of blood cells of the blood of the standard health at the internal area A.

A diagnosis section 8A is provided with the deformability calculation means 81, which calculates the deformability of blood cells by the velocity of blood cells obtained by the image processing section 7A, in addition to the analytical means such as a CPU or the memory means such as a semiconductor memory, and is electrically connected with the image processing section 7A. The diagnosis section 8A calculates the deformability ofblood cells such as a gate number N which will be described later and gate number ratio L of velocities R, as a fluidity ofblood and diagnoses the degree of health ofblood by the deformability. Further the diagnosis section 8A is provided with data which are necessary for judging the degree of health ofblood. The diagnosis section 8A may be constituted in an integrated fashion with the image processing section 7A using a PC or other means.

The blood fluidity measurement method via the blood fluidity measurement apparatus 1 A according to the modified embodiment will be described.

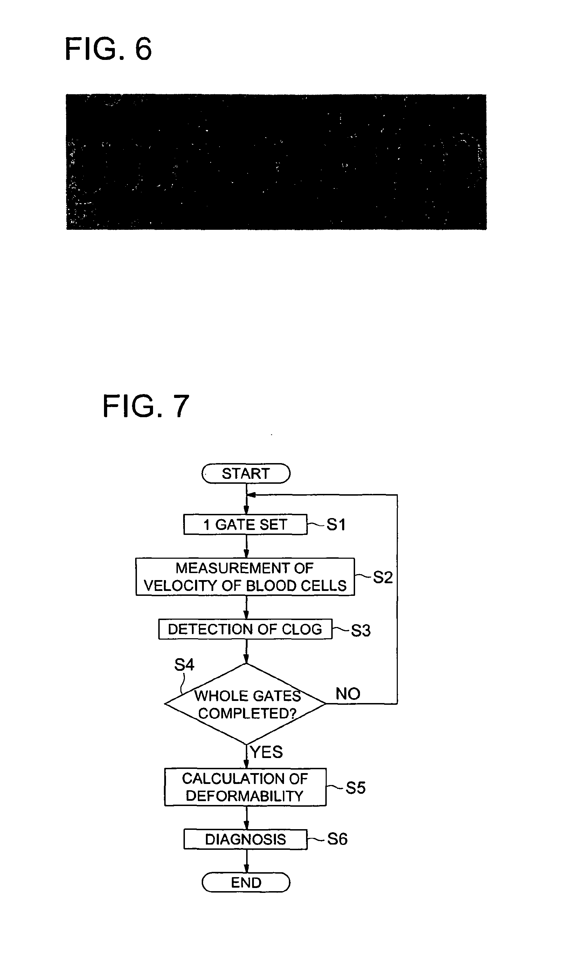

First, steps until a step of taking an image of blood flow after blood is flowed in the filter 2 is same as the embodiment described above. One example of the image ofblood flow taken in the steps is shown in Fig. 6. In the figure, clogs of white blood cells happen at the gate 30 where the color of the area A is light. White blood is easy to clog at the gate 30 because the diameter of white blood cells is larger than the red blood cells.

Next, a process in which the taken images of moving images of blood flow are processed to investigate the fluidity will be described. This process is executed following steps shown in Fig. 7.

First, one of the gates 30 to be inspected is set (S1). Then the velocity ofblood cells at the set gate 30 is calculated by the image processing section 7A (S2).

In calculating the velocity ofblood cells, at least the velocity V_{A} at internal area A of gate 30 is calculated. At this occasion, it is preferable to calculate the velocity of bloods on the center line of the gate 30. For the calculating the velocity, same method as the embodiment described above can be used. The velocity to be calculated can be a value represented by one blood cell for each area or can be a mean value of calculated velocities of a plurality of blood cells.

Following to the calculation of the velocity of blood cells, the clog at gate 30 is detected by the image processing section 7A (S3). This is to detect a state like the state described above in which white blood cells clog at gate 30. When the gate 30 becomes to be this state of clog, the blood flow is blocked and the velocity of the blood flow becomes extremely low. Therefore, the clog can be detected by comparing the velocity V_{A} ofblood cells at the internal area A with a predetermined threshold value. The predetermined threshold value as a preferable value is a half of a velocity V_{S} of blood cells in blood when blood having a standard fluidity flows in the internal area and is, more preferably 5% of the velocity V_{S} of blood cells in blood when blood having a standard fluidity flows in the internal area.

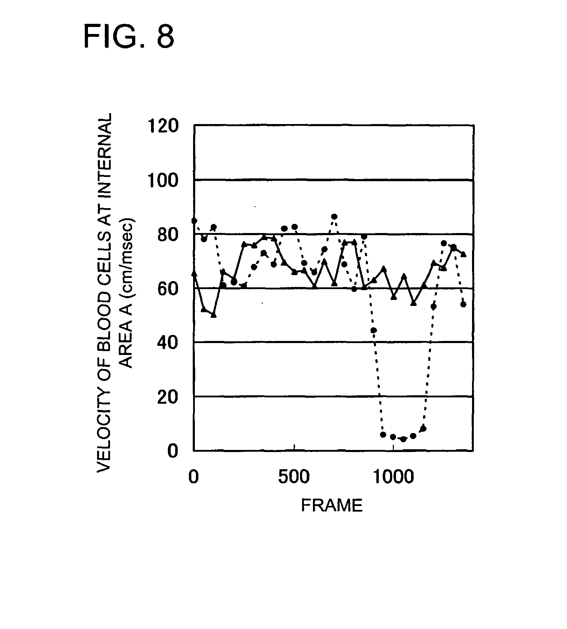

Further the above described predetermined threshold value of 5% of the velocity V_{S} depends on the data of the blood velocity which Fig. 8 shows. Fig. 8 shows a change of the velocity ofblood cells at the internal area A when the standard blood is flown. The solid line and the broken line in the figure are respectively a velocity of different gates, and the horizontal line represents frame numbers of moving image of blood flow. According to the figure, the velocity ofblood cells shown by the solid line keeps approximately constant over all the frames, however the velocity ofblood cells shown by the broken line keeps goes down extremely temporarily. The temporal going down of the velocity of blood cells represents that white blood cells clog at a position where they advance with some extent in the internal area A. Therefore the ratio of the velocity of white blood cells (about 4 cm/ms) when they are clogged, to the velocity (about 70 cm/ms) at the part where blood cells flow healthily can be said threshold value to detect clog.

After completing the detection of clog, steps up to here (S1 through S3) are performed for all gates 30.

Next, the deformability ofblood cells is calculated by the deformability calculation means 81 as a fluidity of blood (S5). Specifically, the gate number N where the clogs happen, and/or the gate number ratio L which is a ratio of the gate number N to the total number of all gates 30 are calculated, as the deformability.

As explained above, according to the blood fluidity measurement apparatus 1A, mainly the deformability of white blood cells can be quantified in a short time, by detecting clogs at the gate 30 and calculating the gate number N and/or the gate number ratio L as the deformability. Further the quantification of the deformability can be achieved only by one or two drops ofblood.

Finally, diagnosis section 8A performs the diagnosis for the deformability (S6). The diagnosis is performed by comparing the gate number N and/or the gate number ratio L obtained as the deformabilities with similar deformabilities of the blood of a standard degree of health. These deformabilities are diagnosed as higher degree of health then a standard, when they are smaller than the similar deformabilities of the blood of the standard degree of health.

As explained above, according to the blood fluidity measurement apparatus 1A, mainly the deformability of white blood cells can be quantified in a short time only with a small quantity of blood, because the velocity V_{A} of blood at the internal area of gate 30 is calculated, the gate number N where the velocity V_{A} is lower than the predetermined threshold value and/or the gate number ratio L which is the ratio of the gate number N to the total number of all gates 30 a as the deformability are calculated by the image processing section 7A.

Further, the present invention is not limited to the above-described embodiments and the modified examples thereof, and naturally it can appropriately be modified.

## Claims

1. A blood fluidity measurement apparatus which measures blood fluidity by flowing blood in a plurality of gates, wherein a width of each of the plurality of gates is formed to be narrower than a diameter of blood cell, the blood fluidity measurement apparatus comprising:
an imaging means which takes an image of blood flow at any two areas of an internal area, an inlet area and an outlet area of the gate, or at each internal area of the plurality of the gates;
a velocity calculation means which calculates a velocity ofblood cells in blood by the image taken by the imaging means; and
a deformability calculation means which calculates a deformability of blood cells as a blood fluidity, by the velocity.

2. The blood fluidity measurement apparatus described in Claim 1, **characterized in that:**
the imaging means takes an image of blood flow in any two areas of an internal area, an inlet area and an outlet area of the gates,
the velocity calculation means calculates velocities of blood cells at the two areas of at least one of the gates; and
the deformability calculation means calculates a difference or a ration between each other of the velocities as the deformability ofblood cells.

3. The blood fluidity measurement apparatus describe in Claim 1, **characterized in that:**
the imaging means takes an image of blood flow at an internal area of each of the plurality of the gates;
the velocity calculation means calculates a velocity of blood cells at the internal area;
the deformability calculation means calculates a number of gates whose velocities are less than or equal to a predetermined threshold value, as the deformability ofblood.

4. The blood fluidity measurement apparatus described in Claim 1, **characterized in that:**
the imaging means takes an image ofblood flow at an internal area of each of the plurality of the gates;
the velocity calculation means calculates a velocity of blood cells in the internal area;
the deformability calculation means calculates a ratio of a number of gates whose velocities are less than or equal to a predetermined threshold value, to a total number of the gates, as the deformability ofblood.

5. The blood fluidity measurement apparatus described in Claim 3 or Claim 4, **characterized in that:**
the deformability calculation means employs a half of a velocity of blood cells in blood when blood having a standard fluidity flows in the internal area, as the predetermined threshold value.

6. The blood fluidity measurement apparatus described in Claim 3 or Claim 4, **characterized in that:**
the deformability calculation means employs a value which is 5% of a velocity ofblood cells in blood when blood having a standard fluidity flows in the internal area, as the predetermined threshold value.

7. A blood fluidity measurement method **characterized in that** the method comprises measuring the blood fluidity using the blood fluidity measurement apparatus described in any one of Claims 1 to 6.
